# EUROPÄISCHE PATENTSCHRIFT

(11) **EP 2 114 354 B1**
(45) Veröffentlichungstag und Bekanntmachung des Hinweises auf die Patenterteilung: **20.06.2018**
(21) Anmeldenummer: 08707435.7
(22) Anmeldetag: 31.01.2008
(51) Int. Cl.: A61Q 17/04, A61K 8/49, A61K 8/02, A61K 8/29, A61K 8/27, A61K 8/19

(54) **LICHTSCHUTZZUBEREITUNG MIT EINER KOMBINATION VON MIKROPIGMENTEN**
LIGHT PROTECTIVE PREPARATION WITH A COMBINATION OF MICROPIGMENTS
PRÉPARATION DE PROTECTION CONTRE LA LUMIÈRE COMPRENANT UNE COMBINAISON DE MICROPIGMENTS

(30) Priorität: 01.02.2007 DE 102007005335
(43) Veröffentlichungstag der Anmeldung: 11.11.2009
(73) Patentinhaber: Beiersdorf AG, 20253 Hamburg (DE); Blatt, Thomas, 22880 Wedel (DE); Wolber, Rainer, 22397 Hamburg (DE); Smuda, Christoph, 25474 Bönningstedt (DE); Clausen, Andreas, 20146 Hamburg (DE); Mundt, Claudia, 8037 Zürich (CH)
(72) Erfinder: BLATT, Thomas, 22880 Wedel (DE); WOLBER, Rainer, 22397 Hamburg (DE); SMUDA, Christoph, 25474 Bönningstedt (DE); CLAUSEN, Andreas, 25436 Tornesch (DE); MUNDT, Claudia, CH-8037 Zürich (CH); REINECKE, Myriam, 22529 Hamburg (DE)
(86) Internationale Anmeldenummer: PCT/EP2008/000746
(87) Internationale Veröffentlichungsnummer: WO 2008/092673

(56) Entgegenhaltungen:
- WO-A2-2007/071584
- DE-A1-102005 010 803
- GB-A- 2 412 866
- GB-A- 2 433 439
- US-A1- 2006 018 846
- POPOV ALEXEY P ET AL: "Effect of size of TiO2 nanoparticles embedded into stratum corneum on ultraviolet-A and ultraviolet-B sun-blocking properties of the skin" 8. Dezember 2005 (2005-12-08), JOURNAL OF BIOMEDICAL OPTICS, SPIE, BELLINGHAM, WA, US, PAGE(S) 64037/1 - 64037/9 , XP002435985 ISSN: 1083-3668 das ganze Dokument

## Beschreibung

Die vorliegende Erfindung betrifft eine kosmetische Zubereitung mit einer Kombination aus anorganischen und organischen UV-Lichtschutzfilterpigmenten.
Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.
Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der deutschen Kosmetikverordnung zusammengefasst.
Eine besondere Form von UV-Lichtschutzfiltersubstanzen stellen die anorganischen Mikropigmente dar. Die UV-Schutzwirkung der Mikropigmente beruht auf den physikalischen Effekten der Reflexion und Lichtstreuung. In kosmetischen Zubereitungen werden als anorganische Mikropigmente meist Pigmente aus Titandioxid, Zinkoxid oder Mischoxiden mit zum Beispiel Eisenoxiden eingesetzt.
Die Vorteile von anorganischen Mikropigmenten als UV-Filtersubstanz in kosmetischen Zubereitungen liegen vor allem darin begründet, dass die Pigmente im Gegensatz zu organischen Lichtschutzfiltern, physikalisch und chemisch besonders stabil sind. Sie neigen selbst bei starker UV-Strahlung nicht zur Zersetzung oder zu Photoreaktionen. Aufgrund der

Tatsache, dass es sich bei den Pigmenten um Feststoffe handelt, besteht keine Gefahr einer übermäßigen Penetration der Filter-in die Haut. Das Auftreten von allergischen Reaktionen ist damit ausgeschlossen.

Nachteilig am Stande der Technik ist jedoch der Umstand, dass anorganische Mikropigmente nur schwer stabil in kosmetische Zubereitungen einzuarbeiten sind. Insbesondere wenn Mikropigmente in höheren Konzentrationen (Konzentrationen über 7 Gewichts-%, bezogen auf das Gesamtgewicht der Zubereitung) eingesetzt werden, bilden sie relativ schnell Pigment-Agglomerate, die aus der Zubereitung ausfallen. Die Lagerstabilität ist also gering. Auch bei der Anwendung auf der Haut führen höhere Konzentrationen von anorganischen Mikropigmenten schnell zur Agglomeration die in Form von weißen Flecken und Streifen ("weißeln") auf der Haut sichtbar und vom Anwender als unästhetisch empfunden werden. Ferner führt die Bildung der Agglomerate zu einer Veränderung im Absorptionsverhalten der Pigmente während der Anwendung. Größere Agglomerate absorbieren überwiegend im Bereich des sichtbaren Lichtes (daher das "weißeln") sowie im UV-A-Bereich, während kleinere Partikel ihr Absorptionsmaximumm im UV-B-Bereich besitzen. Darüber hinaus ist die UV-Lichtschutzwirkung von größeren Teilchen deutlich geringer als die von Mikropigmenten. Nach dem Stande der Technik ist es also nahezu unmöglich, kosmetische UV-Lichtschutzzubereitungen auf der Basis von anorganischen Mikropigmenten herzustellen, die während der Dauer ihrer Anwendung ein stabiles und ausgewogenes Absorptionsspektrum aufweisen.

Nach dem Stand der Technik lassen sich diese Probleme allein dadurch begrenzen, dass als kosmetische Grundlage der Sonnenschutzzubereitung eine Wasser-in-Öl-Emulsion (W/O-Emulsion) eingesetzt werden. W/O-Emulsionen weisen aber, da ihre äußere Phase von der Ölphase gebildet wird, in der Regel ein eher ölig-fettiges Hautgefühl auf. W/O-Emulsionen mit einem erhöhten Gehalt an anorganischen Mikropigmenten wirken darüber hinaus zäh und lassen sich nur schwer auf der Haut verteilen. Um die Verteilbarkeit solcher Zubereitungen zu verbessern, müssen den Formulierungen Silikonöle zugesetzt werden, was häufig nicht erwünscht ist.

In die kosmetisch-sensorisch attraktiveren Öl-in-Wasser-Emulsionen (O/W-Emulsionen) ließen sich bisher keine größeren Mengen an anorganischen Mikropigmenten einarbeiten, ohne dass die beschriebenen Nachteile (z.B. mangelnde Stabilität) zu vermeiden gewesen wären. Es ließen sich allenfalls Zubereitungen mit geringem Lichtschutzfaktor stabil formulieren. O/W-Lichtschutzemulsionen mit hohem Lichtschutzfaktor ließen sich, nach dem Stand der Technik, nur mit Hilfe flüssiger oder gelöster organischer Lichtschutzfilter erreichen. Neben dem Problem der Hautpenetration dieser Filter, weisen die Zubereitungen insbesondere bei höheren Filterkonzentrationen, ein fettig-klebriges Hautgefühl auf.

Aufgabe der vorliegenden Erfindung war es daher, die Nachteile des Standes der Technik zu beseitigen und kosmetische Lichtschutzzubereitungen mit hohem Lichtschutzfaktor auf wässriger Basis oder auf der Basis von Öl-in-Wasser-Emulsionen (O/W-Emulsionen) zu entwickeln, deren UV-Filtersystem auf Mikropigmenten basiert. Diese Zubereitungen sollten insbesondere lager- und transportstabil sein und eine Agglomeration der Mikropigmente während der Lagerung und der Anwendung wirkungsvoll unterdrücken. Die Formulierungen sollten ferner bei Ihrer Anwendung auf der Haut ein über einen längeren Anwendungszeitraum stabiles Absorptionsspektrum mit einer ausgewogenen UV-A/UV-B-Absorptionsbalance aufweisen.

Überraschend gelöst werden die Aufgaben durch eine kosmetische Zubereitung, die in Form einer wässrigen Zubereitung oder einer O/W-Emulsion vorliegt, enthaltend
a) partikuläre anorganische UV-Lichtschutzfilterpigmente mit einer durchschnittlichen Partikelgröße von 10 bis 500 nm,
b) 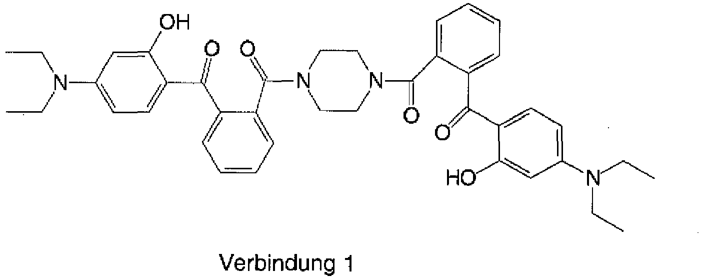 und
c) eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, β-Alanin, Tocopherylacetat, Harnstoff; Hyaluronsäure; Dihydroxyaceton; 8-Hexadecen-1,16-dicarbonsäure und/oder Licochalcon A.

Die erfindungsgemäßen Zubereitungen zeigen einen überraschend breiten UV-Absorptionsbereich, hohe spezifische Absorption in diesem Bereich, Photo- und Thermostabilität, Hautverträglichkeit (keine reizenden und toxischen Wirkungen auf der Haut), gutes Hautgefühl und gute Haftung auf der Haut, Wasserfestigkeit sowie gute Verträglichkeit mit anderen kosmetischen Substanzen.

Zwar weisen beschreiben die DE 10162842, DE19923645, DE19933461, DE10040481, DE10063345, DE10100409, DE10008896, DE10161884, DE10226353, DE10140546, DE10135024, DE10154111, DE10260877, DE10141473, DE10141474, DE10247357, EP1302197, EP1093798, EP1093797, EP1093796. EP1064922, EP893119 und EP1068866 kosmetische Zubereitungen mit einem Gehalt an anorganischen UV-Filterpigmenten, doch konnten diese Schriften nicht den Weg zur vorliegenden Erfindung weisen.

Darüber hinaus kennt der Stand der Technik die US 2006/018846, GB 2412866, GB 2433439, DE 1022005010803 sowie Popov Alexy P. et al. "Effect of size of TiO₂ nanoparticles embedded into stratum corneum on ultraviolet-A and ultraviolet-B sunblocking properties of the skin", 8. Dezember 2005, Journal of Biomedical Optics, Spie, Bellingham, WA, US, pages 64037/1-64037/9, XP002435985, ISSN: 1083-3668, die ebenfalls nicht den Weg zur vorliegendne Erfindung weisen konnten.

Die erfindungsgemäßen Zubereitungen weisen eine überraschend hohe Wasserfestigkeit sowie eine angenehme Sensorik auf der Haut auf.

### Messmethoden

Bestimmung des erfindungsgemäßen Lichtschutzfaktors erfolgte wie in der DIN 67501 Sep. 1999 angeben, nach der gemessen wird.

Die Partikelgröße der partikulären anorganischen UV-Lichtschutzfilterpigmente wurde mittels Rasterelektronenmikroskopie (REM) bestimmt.

Die im Rahmen der vorliegenden Schrift aufgeführten Viskositätswerte der Zubereitungen und Einzelsubstanzen wurden mit Hilfe eines Viskosimeters des Typs Viskotester VT 02 der Gesellschaft Haake ermittelt (Temperatur: 20°C, Spindeldurchmesser 24 mm, Rotorgeschwindigkeit 62 1/min nach 30s).

Unter "Normalbedingungen" werden erfindungsgemäß Temperaturen von 20 °C bei einem Druck von 1,013 bar verstanden.

Es ist erfindungsgemäß vorteilhaft, wenn die erfindungsgemäße Zubereitung partikuläre anorganische Lichtschutzfilterpigmente in einer Konzentration von 0,1 bis 15 Gewichts-% und bevorzugt in einer Konzentration von 0,5 bis 10 Gewichts-%, jeweils bezogen auf das Gesamtgewicht der Zubereitung, enthält.

Es ist erfindungsgemäß vorteilhaft, wenn die erfindungsgemäße Zubereitung die Verbindung 1 in Konzentration von 0,25 bis 10,0 Gewichts-% und bevorzugt in einer Konzentration von 0,5 bis 5,0 Gewichts-%, jeweils bezogen auf das Gesamtgewicht der Zubereitung, enthält.

Es ist erfindungsgemäß vorteilhaft, Verbindung 1 in Form einer wässrigen Dispersion zuzusetzen. Derartige Dispersionen enthalten Verbindung 1 in der Regel in einem Gehalt von 45 bis 50 Gewichts-%, bezogen auf das Gesamtgewicht der Dispersion. Als Dispergierhilfen enthalten solche Dispersionen in der Regel die üblichen Dispergiermittel, beispielsweise Plantacare 2000 UP (Cognis), Amphisol K (DSM), Texapon K14S Spezial (Cognis), Rhodicare S (C.H. Erbslöh KG), Silfoam SE2 (Wacker), Butylenglycol, Zitronensäure, NaOH.

Es ist dabei vorteilhaft im Sinne der vorliegenden Erfindung, wenn die partikulären organischen UV-Lichtschutzfilterpigmente einen durchschnittlichen Partikeldurchmesser von 10 bis 300 nm und besonders bevorzugt von 10 bis 200 nm aufweisen.

Die erfindungsgemäßen Mikropigmente können erfindungsgemäß vorteilhaft in einer wässrigen Dispersion vorliegen. Als erfindungsgemäß vorteilhafte Dispergierhilfen können beispielsweise C8-C16 Alkylpolyglucosid und/oder amphiphile Polymere, wie sie in der EP 1093796 B1 beschrieben sind, eingesetzt werden

Es ist dabei erfindungsgemäß von Vorteil, wenn die anorganischen UV-Lichtschutzfilterpigmente gewählt werden aus der Gruppe der folgenden Pigmente:
Vorteilhafte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden, sowie das Sulfat des Bariums (BaSO₄). Es ist dabei erfindungsgemäß bevorzugt, wenn als partikuläre anorganische UV-Lichtschutzfilterpigmente Titandioxid, Zinkoxid und/oder Eisenoxid eingesetzt werden.

Erfindungsgemäß besonders bevorzugt ist es, wenn als anorganische Lichtschutzfilterpigmente Titandioxid eingesetzt werden.

Erfindungsgemäß vorteilhafte Ausführungsformen der vorliegenden Erfindung sind dadurch gekennzeichnet, dass die partikulären anorganischen UV-Lichtschutzfilterpigmente oberflächenbeschichtet sind.

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form kommerziell erhältlicher öliger oder wäßriger Vordispersionen zur Anwendung kommen. Diesen Vordispersionen können vorteilhaft Dispergierhilfmittel und/oder Solubilisationsvermittler zugesetzt sein.

Die erfindungsgemäßen Titandioxide zeichnen sich durch eine Primärpartikelgröße zwischen 10 nm bis 200 nm aus, wobei Partikelgrößen von 10 nm bis 100 nm erfindungsgemäß bevorzugt sind.

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eusolex TS | Alumina, Stearinsäure | - | Merck KgaA |
| Eusolex T-AVO | Silica | | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |
| Titandioxid T-817 (Eisen/TitanMisch oxid) | | | Degussa |

Im Sinne der vorliegenden Erfindung sind besonders bevorzugte Titandioxide das MT-100 Z und MT-100 TV von Tayca Corporation, Eusolex T-2000 und Eusolex T-AVO von Merck und das Titandioxid T 805 von Degussa und das Eisen/Titandmischoxid Titandioxid T817 von Degussa.

Zinkoxide können im Sinne der vorliegenden Erfindung auch in Form kommerziell erhältlicher öliger oder wässriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Erfindungsgemäß vorteilhafte Ausführungsformen der vorliegenden Erfindung sind dadurch gekennzeichnet, dass mindestens zwei unterschiedliche Typen von anorganischen UV-Lichtschutzfilterpigmenten eingesetzt werden. Dabei ist es erfindungsgemäß bevorzugt, wenn Mischungen aus Titandioxid- und Zinkoxidpigmenten eingesetzt werden.

Die erfindungsgemäß bevorzugten Ausführungsformen der vorliegenden Erfindung sind, dadurch gekennzeichnet, dass die partikulären anorganischen UV-Lichtschutzfilterpigmente oberflächenbeschichtet sind.

Erfindungsgemäß besonders bevorzugt ist es, hydrophob modifizierte Titandioxidpigmente mit amphiphilen Zinkoxidpigmenten zu kombinieren.

Erfindungsgemäß vorteilhafte Ausführungsformen der vorliegenden Erfindung sind dadurch gekennzeichnet, dass die Zubereitung weitere organische UV-Lichtschutzfilterpigmente gewählt aus der Gruppe der Verbindungen 2,2'-Methylen-bis-(6-(2H-benzotriazol-2-yl)-4-(1,1,3,3-tetramethylbutyl)-phenol); 2,4,6-Tris-(biphenyl)-1,3,5-triazin; 2,4,6-Tris-(terphenyl)-1,3,5-triazin; 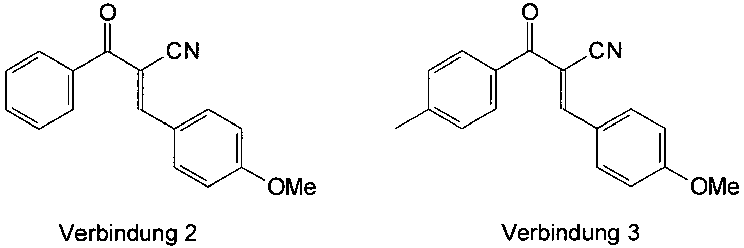 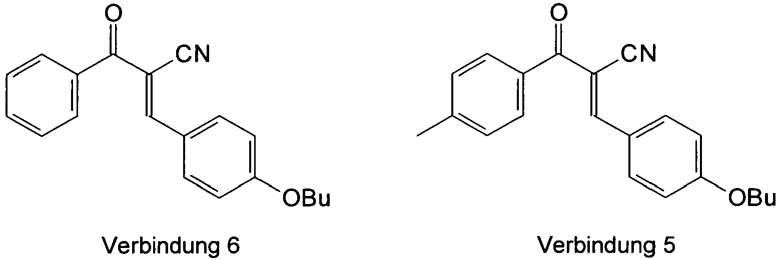 enthält.

Derartige zusätzliche UV-Filter liegen in der Zubereitung erfindungsgemäß vorteilhaft in einer Konzentration von 0,1 bis 20 Gewichts-%, bezogen auf das Gesamtgewicht der Zubereitung in der Zubereitung vor.

In einer erfindungsgemäß bevorzugten Ausführungsform ist die erfindungsgemäße Zubereitung frei von in der Öl- oder Wasserphase löslichen organischen UV-Lichtschutzfiltern.

Es ist erfinsdungsgemäß vorteilhaft, wenn die erfindungsgemäße Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat , Natriumcetearylsulfat, Kaliumcetylphosphat enthält.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

Es ist erfindungsgemäß bevorzugt, wenn die erfindungsgemäße Zubereitung frei ist von in der Öl- oder Wasserphase löslichen oder flüssigen organischen UV-Lichtschutzfiltern. Die erfindungsgemäße Emulsion kann aber auch ein oder mehrere der in der Kosmetik bekannten, in der Öl- oder Wasserphase löslichen oder flüssigen organischen UV-Lichtschutzfilter enthalten.

Es ist vorteilhaft im Sinne der vorliegenden Erfindung, wenn die Zubereitung einen Lichtschutzfaktor von mindestens 10 aufweist und erfindungsgemäß bevorzugt, wenn die Zubereitung einen Lichtschutzfaktor von mindestens 15 aufweist.

Erfindungsgemäß vorteilhafte Ausführungsformen der vorliegenden Erfindung sind ferner dadurch gekennzeichnet, dass die Zubereitungen eine Viskosität von 200 bis 15000 mPas und besonders bevorzugt eine Viskosität von 1500 bis 12000 mPas aufweisen.

Die Wasserphase der erfindungsgemäßen Zubereitung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Propandiol Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination. Erfindungsgemäß bevorzugt können auch Verdicker, wie Permulen TR 1, TR 2, Carbopol 1328, Aristoflex AVC eingesetzt werden.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an zyklischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 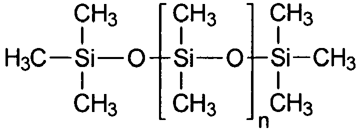 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), zyklische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).
Erfindungsgemäß vorteilhaft enthält die erfindungsgemäße Zubereitung unter Normalbedingungen flüssige Öle in einer Konzentration von weniger als 10 Gewichts-%, bezogen auf das Gesamtgewicht der Zubereitung.

Erfindungsgemäß bevorzugte Ausführungsformen der vorliegenden Erfindung sind dadurch gekennzeichnet, dass die Zubereitung einen Gehalt an bei unter Normalbedingungen flüssigen Ölen von unter 5 Gewichts-%, bezogen auf das Gesamtgewicht der Zubereitung, aufweist. In einer besonders bevorzugten Ausführungsform der vorliegenden Erfindung sind die erfindungsgemäßen Zubereitungen frei von derartigen Ölen.

Die erfindungsgemäßen Zubereitungen können ferner vorteilhaft auch Selbstbräunungssubstanzen enthalten, wie beispielsweise Dihydroxyacteon und/oder Melaninderivate in Konzentrationen von 1 Gew.-% bis zu 10 Gew.-%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und/oder Nylon 12 und/oder Covabead LH 85 und/oder Mearlmica SVA.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder - SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Erfindungsgemäß vorteilhaft können die erfindungsgemäßen Zubereitungen zum Schutz vor farblichen Veränderungen von Hauttätowierungen (Tatoos) verwendet werden.

Zur Anwendung werden die erfindungsgemäßen kosmetischen Zubereitungen in der für Kosmetika üblichen Weise auf die Haut und/oder die Haare in ausreichender Menge aufgebracht.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

**W/O Emulsion**

| **Emulsion** | **1** | **2** | **3** | **4** | **5** | **6** |
|---|---|---|---|---|---|---|
| Polyglyceryl-2 Dipolyhydroxystearat | 3 | 5 | 3 | | | |
| PEG-30 Dipolyhydroxystearat | | | **2** | **3** | **4** | 5 |
| Natrium-Stärke Octenylsuccinat | 0,5 | 0,4 | 0,6 | 0,3 | 0,5 | 1 |
| Glycin | 0,3 | 0,3 | 0,5 | 0,4 | | |
| Alkohol | 2 | 5 | 2 | 5 | 4 | 3 |
| Magnesiumsulfat | 0,2 | 0,3 | 0,3 | 0,4 | 0,5 | 0,2 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Mineralöl | | 6 | 4 | | | 5 |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylenglykol | | | 1 | | 5 | 3 |
| Glycerin | 3 | 5 | 7 | 10 | 3 | 3 |
| C18-38 Säuretriglycerid | 0,5 | | 1 | | 1 | |
| Titandioxid T 805® | 4 | | | 2 | | |
| Titandioxid T 817® | | 3 | | | | |
| Eusolex T-AVO® | | | 1 | | | 1,5 |
| Titandioxid MT-100 Z® | | | | | 2 | |
| Zinkoxid NDM® | 2 | | | | | |
| Izinkoxid Z-Cote HP1® | | | | | 1 | |
| Eusolex T-2000® | | 2 | | | | |
| 2,2 -Methylen-bis-(6-(2H-benzotriazol-2-yl)-4-(1,1,3,3-tetramethylbutyl)-phenol) | 2 | | | | | |
| 2,4,6-Tris-(biphenyl)-1,3,5-triazin | 1 | | 2 | | | |
| Mikron. Hydroxyphenyl Benzophenon (Verbindung 1) | 2 | 1 | 3 | 4 | 2 | 2,5 |
| Taurin | 0,1 | | | 0,5 | 0,2 | |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

| **Emulsion** | **7** | **8** | **9** | **10** | **11** | **12** |
|---|---|---|---|---|---|---|
| Polyglyceryl-3 Diisostearat | 1,5 | 1,5 | | | | 1,5 |
| Polyglyceryl-2 Dipolyhydroxystearat | | 2 | 2 | 2 | | |
| Cetyl Dimethicone Copolyol | | | 4 | 2,5 | 3 | |
| PEG 45 Dodecyl Glycol Copolymer | | | | 1 | | |
| PEG-30 Dipolyhydroxystearat | | | | | 3 | 2 |
| Natrium-Stärke Octenylsuccinat | 0,3 | 0,3 | 0,5 | 0,4 | | |
| Glycin | 2 | 5 | 2 | 5 | 4 | 3 |
| Alkohol | 0,2 | 0,3 | 0,3 | 0,4 | 0,5 | 0,2 |
| Magnesiumsulfat | 0,2 | 0,3 | 0,3 | 0,4 | 0,5 | 0,2 |
| C₁₂₋₁₅ Alkyl Benzoat | 5 | 3 | | | 5 | |
| Butylenglycol Dicaprylat/Dicaprat | 5 | | | 5 | 3 | 2 |
| Dicaprylyl Ether | | | | | 2 | |
| Diethylhexyl naphthalat | 2 | | | | | |
| Dicaprylcaprat | | 2 | | | 2 | 2 |
| Cyclomethicon | 5 | | 5 | 15 | | |
| Isohexadecan | | 5 | | | | |
| Mineralöl | 3 | 1 | | | 2 | 5 |
| Propylenglykol | | | 4 | | | |
| Glycerin | 5 | 7 | 3 | 5 | 6 | 8 |
| C18-38 Säuretriglyceride | 0,5 | | 1 | | 1 | |
| Titandioxid T 805® | 1,5 | | | 1 | 8 | 2 |
| Titandioxid T 817® | | 2 | | | | |
| Eusolex T-AVO® | | | 2 | | | |
| Titandioxid MT-100 Z® | | | 2 | 1 | | |
| Zinkoxid NDM® | | | | | 7 | |
| Zinkoxid Z-Cote HP1® | | | | | | 1 |
| Eusolex T-2000® | | 2 | | | | |
| 2,2'-Methylen-bis-(6-(2H-benzotriazol-2-yl)-4-(1,1,3,3-tetramethylbutyl)-phenol) | | | | | | 3 |
| 2,4,6-Tris-(biphenyl)-1,3,5-triazin | | 1 | | | | 2 |
| Mikron. Hydroxyphenyl Benzophenon (Verbindun 1) | 2 | 1 | 3 | 0,5 | 3 | 2,5 |
| Kreatinin | 0,1 | 0,01 | 0,05 | | | |
| Kreatin | 0,5 | 0,2 | 0,1 | | | |
| Vitamin E Acetat | 0,2 | | | 0,5 | 0,5 | 0,5 |
| Na₂H₂EDTA | 0,1 | | 0,2 | | | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

| **Emulsion** | **13** | **14** | **15** | **16** | **17** | **18** |
|---|---|---|---|---|---|---|
| Polyglyceryl-2 Dipolyhydroxystearat | 3 | 5 | 3 | | | |
| PEG-30 Dipolyhydroxystearat | | | 1 | 3 | 4 | 5 |
| Natrium-Stärke Octenylsuccinat | 0,5 | 0,4 | 0,6 | 0,3 | | 1 |
| Glycin | 0,3 | 0,3 | 0,5 | 0,4 | | |
| Alkohol | 2 | 5 | 2 | | 4 | 3 |
| Magnesiumsulfat | 0,2 | | 0,3 | 0,4 | 0,5 | 0,2 |
| C₁₂₋₁₅ Alkyl Benzoat | 5 | 3 | | | 5 | |
| C₁₂₋₁₃ Alkyl Tartrat | | 2 | | | | 5 |
| Butylenglycol Dicaprlat/Dicaprat | 5 | | | | 3 | 3 |
| Dicaprylyl Ether | | | | | 2 | |
| Mineralöl | | 4 | | 8 | | 2 |
| Octyldodecanol | 2 | | | | | |
| Dicaprylcaprat | | 2 | | | 5 | 8 |
| Cyclomethicon | 5 | | 5 | 10 | | |
| Dimethicon | | | | 5 | | 5 |
| Isohexadecan | | 1 | | | | |
| Butylenglycol | 5 | 8 | | | | 3 |
| Propylenglykol | | | 1 | | 5 | 3 |
| Glycerin | 3 | 5 | 7 | 10 | 3 | 3 |
| C18-38 Säuretriglyceride | 0,5 | | 1 | | 1 | |
| Titandioxid T 805® | 2 | | 3 | | | 2 |
| Titandioxid MT-100 Z® | | 1 | | 1 | | |
| Zinkoxid NDM® | | | | | 1 | |
| Zinkoxid Z-Cote HP1® | | | 1 | | | |
| 2,2'-Methylen-bis-(6-(2H-benzotriazol-2-yl)-4-(1,1,3,3-tetramethylbutyl)-phenol) | | | | 4 | | |
| 2,4,6-Tris-(biphenyl)-1,3,5-triazin | | | 2 | | | 5 |
| Mikron. Hydroxyphenyl Benzophenon (Verbindung 1) | 2 | 1 | 3 | 0,5 | 3 | 2,5 |
| Ethylhexylmethoxycinnamat | | 6 | 5 | | 2 | 1 |
| Phenylbenzimidazol Sulfonsäure | | 0,5 | 2 | | 1 | |
| Homosalat | 5 | | | 7 | 1 | |
| Butyl Methoxydibenzoylmethan | | 3 | | | | 2 |
| Bis-Ethylhexyloxyphenol Methoxyphenyltriazin | | 2 | 3 | | | 1 |
| Octylsalicylat | | | | 5 | 1 | |
| Parsol® SLX | | | | | 1 | |
| Taurin | 0,1 | | | 0,5 | 0,2 | |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

**O/W Emulsion**

| **Emulsion** | **1** | **2** | **3** | **4** | **5** | **6** |
|---|---|---|---|---|---|---|
| Glyceryl Stearat Citrat | 2,5 | 2 | 3 | | | |
| Sorbitanstearat | 0,5 | | | 2 | 1,5 | 2 |
| Polyglyceryl-3 Methylglycose Distearat | | | | 2,5 | 3 | 3 |
| Polyglyceryl-2 Dipolyhydroxystearat | | 0,8 | | | | 0,5 |
| Cetearylalkohol | | | | 1 | | |
| Stearylalkohol | 2 | | | | | 2 |
| Cetylalkohol | | 1 | | | 3 | |
| Acrylates/C₁₀₋₃₀ Alkyl Acrylat Crosspolymer | | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylenglykol | | | 1 | | 5 | 3 |
| Glycerin | 3 | 5 | 7 | 10 | 3 | 3 |
| C18-38 Säuretriglyceride | 0,5 | | 1 | | 1 | |
| Titandioxid T 805® | 1 | | | 2 | | |
| Eusolex T-AVO® | | | 4 | | | 2 |
| Titandioxid MT-100 Z® | | | | 2 | | |
| Zinkoxid NDM® | 2 | | | | | |
| Zinkoxid Z-Cote HP1® | | | | | | 1 |
| Eusolex T-2000® | | 5 | | | | |
| 2,2'-Methylen-bis-(6-(2H-benzotriazol-2-yl)-4-(1,1,3,3-tetramethylbutyl)-phenol) | | | | | | 2 |
| Mikron. Hydroxyphenyl Benzophenon (Verbindung 1) | 2 | 1 | 5 | 0,5 | 3 | 1,5 |
| Taurin | 0,1 | 0,2 | | 0,5 | 0,2 | |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

| **Emulsion** | **7** | **8** | **9** | **10** | **11** | **12** |
|---|---|---|---|---|---|---|
| Ceteareth-20 | 1 | 1,5 | 1 | | | |
| Sorbitanstearat | | | 0,5 | | 0,5 | |
| Glyceryl Stearat SE | | | | 1 | 1 | 1,5 |
| Emulgade F® | | | | 2,5 | 2,5 | 3 |
| Cetearylalkohol | | | | 1 | | |
| Stearylalkohol | | | | | 1,5 | |
| Cetylalkohol | | | 0,5 | | | 2 |
| Acrylates/C₁₀₋₃₀ Alkyl Acrylat Crosspolymer | 0,2 | 0,4 | 0,3 | 0,1 | | |
| Carbomer | | | | | 0,3 | |
| Xanthan Gum | | | | 0,4 | | 0,4 |
| C₁₂₋₁₅ Alkyl Benzoat | 5 | 3 | | | 5 | |
| 2-Phenylbenzoat | | 2 | | | | |
| Butylenglycol Dicaprylat/Dicaprat | 5 | | | | 3 | 2 |
| Dicaprylyl Ether | | | | | 2 | |
| Dicaprylcaprat | | 2 | | | 2 | 2 |
| Cyclomethicon | 5 | | 5 | 10 | | |
| Isohexadecan | | | | 5 | | |
| Mineralöl | | 1 | | | | |
| Propylenglykol | | | 4 | | | |
| Glycerin | 5 | 7 | 3 | 5 | 6 | 8 |
| C18-38 Säuretriglyceride | 0,5 | | 1 | | 1 | |
| Titandioxid T 805® | 1 | | | | 1 | |
| Eusolex T-AVO® | | | 5 | | | 2 |
| Titandioxid MT-100 Z® | | | 1 | | | |
| Zinkoxid NDM® | 2 | | | | | |
| Izinkoxid Z-Cote HP1® | | | | | 4 | 1 |
| Eusolex T-2000® | | 5 | | | | |
| 2,2'-Methylen-bis-(6-(2H-benzotriazol-2-yl)-4-(1,1,3,3-tetramethylbutyl)-phenol) | 2 | | | 2 | | 4 |
| 2,4,6-Tris-(biphenyl)-1,3,5-triazin | 1 | 2,5 | 4 | | 1 | |
| Mikron. Hydroxyphenyl Benzophenon (Verbindung 1) | 2 | 1,5 | 1,5 | 4 | 3 | 2,5 |
| Methylpropandiol | 0,5 | 1,4 | 0,75 | | 0,5 | 1,5 |
| Kreatinin | 0,1 | 0,01 | 0,05 | | | |
| Kreatin | 0,5 | 0,2 | 0,1 | | | |
| Vitamin E Acetat | 0,2 | | | 0,5 | 0,5 | 0,5 |
| Na₂H₂EDTA | 0,1 | | 0,2 | | | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

| **Emulsion** | **13** | **14** | **15** | **16** | **17** | **18** |
|---|---|---|---|---|---|---|
| Glycerylstearat SE | | 2 | | 2 | | |
| Glycerylstearat | 2 | | 2 | | | |
| PEG-40 Stearat | | | 2 | | | |
| PEG-100 Stearat | | | | 2,5 | | |
| Ceteareth-20 | | | | | 0,5 | |
| Glyceryl Stearat Citrat | | | | | | 0,4 |
| Stearinsäure | 3 | 2 | | | | |
| Cetearylalkohol | | 2 | 2 | | | |
| Stearylalkohol | 0,5 | | 2 | | | |
| Cetylalkohol | 3 | | | 2 | | |
| Acrylates/C₁₀₋₃₀ Alkyl Acrylat Crosspolymer | | | 0,2 | | 0,4 | 0,3 |
| Carbomer | | 0,3 | | 0,3 | 0,3 | 0,4 |
| Xanthan Gum | | 0,3 | 0,4 | | | |
| C₁₂₋₁₅ Alkyl Benzoat | 5 | | | | 5 | |
| Panthenol | 0,5 | 1,25 | 0,75 | | 1,5 | 2,0 |
| Butylenglycol Dicaprylat/Dicaprat | | 5 | | 4 | | |
| Dicaprylyl Ether | | 2 | | | 3 | |
| Diethylhexylnaphthalat | 3 | | | 3 | | |
| Cyclomethicon | 2 | | 10 | 2 | | 5 |
| Isohexadecan | | | | 2 | 3 | |
| Mineralöl | | | | | 3 | |
| Propandiol | | 3 | | 5 | | |
| Glycerin | 3 | 5 | 10 | 7 | 4 | 6 |
| Titandioxid T 805® | 2 | 1 | 3 | 2 | 1 | 4 |
| Mikron. Hydroxyphenyl Benzophenon (Verbindung 1) | 2 | 1 | 1,5 | 4 | 5 | 0,5 |
| Ethylhexylmethoxycinnamat | | 6 | 5 | | 2 | 1 |
| Phenylbenzimidazol Sulfonsäure | | 0,5 | 2 | | 1 | |
| Homosalat | 5 | | | 7 | 1 | |
| Butyl Methoxydibenzoylmethan | | 3 | | | | 2 |
| Bis-Ethylhexyloxyphenol Methoxyphenyltriazin | | 2 | 3 | | | 1 |
| Octylsalicylat | | | | 5 | 1 | |
| Parsol® SLX | 4 | | | | | |
| PVP/Hexadecen Copolymer | 0,5 | | 1 | | 0,8 | |
| Coenzym Q 10 | 0,2 | 0,02 | | 0,3 | | |
| (Vitamin E Acetat | 0,2 | | 0,3 | | 0,8 | 0,5 |
| Na₂H₂EDTA | 0,1 | | | | | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

| **Emulsion** | **19** | **20** | **21** | **22** | **23** | **24** |
|---|---|---|---|---|---|---|
| Polyglyceryl-3- Methylglucose Distearat | 3,0 | 4,0 | 2,5 | 1,9 | 4,50 | 2,5 |
| Cetyl Alkohol | 1,0 | | 2,00 | | 4,50 | 2,00 |
| Mikron. Hydroxyphenyl Benzophenon (Verbindung 1) | 2,0 | 1,0 | 1,5 | 4,0 | 3,0 | 2,5 |
| Titandioxid T 805 | 1,00 | 3,00 | 2,00 | 4,00 | 2,50 | 1,50 |
| C12-15 Alkyl Benzoat | | | 3,00 | 3,00 | 1,00 | |
| Hydriertes Kokosfettsäureglycerid | 1,00 | 1,00 | 3,00 | | | 3,00 |
| Dicaprylylether | 5,00 | 2,00 | 6,00 | | | 3,00 |
| Octyldodecanol | 6,00 | 5,00 | 4,00 | 1,00 | 2,00 | |
| Butylenglycol Dicaprylat/ Dicaprat | 2,00 | 2,00 | | | | |
| Caprylsäure/Caprinsäuretriglycerid | | | | 2,00 | 1,50 | |
| Dimethicon | | | 2,00 | | | |
| Cyclomethicon | 2,00 | 1,00 | | 3,00 | | |
| PVP Hexadecen Copolymer | 0,50 | 0,50 | | | | |
| Glycerin | 8,00 | 6,00 | 5,00 | 3,00 | 3,00 | 10 |
| Xanthangummi | 0,40 | 0,40 | 0,25 | 0,30 | 0,10 | |
| Nachtkerzenöl | | 1,00 | | | | |
| Pentandiol | 0,05 | | 2,0 | | 1,5 | |
| Panthenol | | 0,1 | | 1,5 | 0,5 | 0,75 |
| Vitamin E Acetat | 0,50 | | 0,30 | 0,40 | 1,40 | |
| Dihydroxyaceton | | 2,00 | | 5,00 | 4,00 | |
| Methylparaben | | | | 0,30 | 0,30 | |
| Phenoxyethanol | 0,40 | 0,40 | 0,35 | 0,50 | 0,50 | 0,50 |
| EDTA | 1,00 | 1,00 | 1,00 | | 1,00 | 1,00 |
| Insekt Repellent 3535 | | | | 4,00 | 5,00 | 5,00 |
| Parfüm | 0,40 | 0,40 | 0,40 | 0,40 | 0,40 | 0,40 |
| Wasser | ad 100 | ad 100 | ad 100 | ad 100 | ad 100 | ad 100 |
| Neutralisationsmittel (Natriumhydroxid, Kaliumhydroxid) | qs | qs | qs | qs | qs | qs |
| pH-Wert | 5,0-7,0 | 5,0-7,0 | 5,0-7,0 | 5,0-7,0 | 5,0-7,0 | 5,0-7,0 |

## Patentansprüche

1. Kosmetische Zubereitung, die in Form einer wässrigen Zubereitung oder einer O/W-Emulsion vorliegt, enthaltend
a) partikuläre anorganische UV-Lichtschutzfilterpigmente mit einer durchschnittlichen Partikelgröße von 10 bis 500 nm,
b) 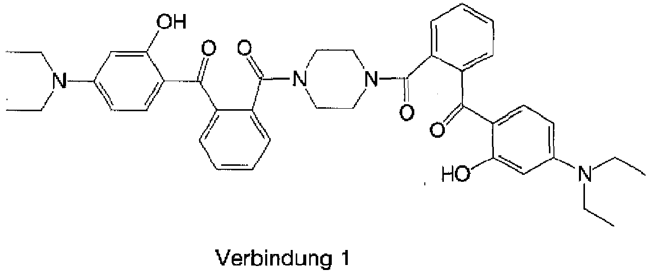 und
c) eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, β-Alanin, Tocopherylacetat, Harnstoff; Hyaluronsäure; Dihydroxyaceton; 8-Hexadecen-1,16-dicarbonsäure und/oder Licochalcon A.

2. Zubereitung nach Anspruch 1, **dadurch gekennzeichnet, dass** die Zubereitung partikuläre anorganische Lichtschutzfilterpigmente in einer Konzentration von 0,5 bis 10 Gewichts-%, bezogen auf das Gesamtgewicht der Zubereitung, enthält.

3. Kosmetische Zubereitung nach einem der vorhergehenden Ansprüche, **dadurch gekennzeichnet, dass** die Zubereitung die Verbindung 1 in einer Konzentration von 0,25 bis 10 Gewichts-%, bezogen auf das Gesamtgewicht der Zubereitung, enthält.

4. Kosmetische Zubereitung nach einem der vorhergehenden Ansprüche, **dadurch gekennzeichnet, dass** als anorganische Lichtschutzfilterpigmente Titandioxid, Zinkoxid und/oder Eisenoxid eingesetzt werden.

5. Zubereitung nach einem der vorhergehenden Ansprüche, **dadurch gekennzeichnet, dass** als anorganische Lichtschutzfilterpigmente Titandioxid eingesetzt wird.

6. Zubereitung nach einem der vorhergehenden Ansprüche, **dadurch gekennzeichnet, dass** die partikulären anorganischen UV-Lichtschutzfilterpigmente oberflächenbeschichtet sind.

7. Zubereitung nach einem der vorhergehenden Ansprüche, **dadurch gekennzeichnet, dass** die Zubereitung weitere organische UV-Lichtschutzfilterpigmente gewählt aus der Gruppe der Verbindungen 2,2'-Methylen-bis-(6-(2H-berzotriazol-2-yl)-4-(1,1,3,3-tetramethylbutyl)-phenol); 2,4,6-Tris-(biphenyl)-1,3,5-triazin; 2,4,6-Tris-(terphenyl)-1,3,5-triazin; 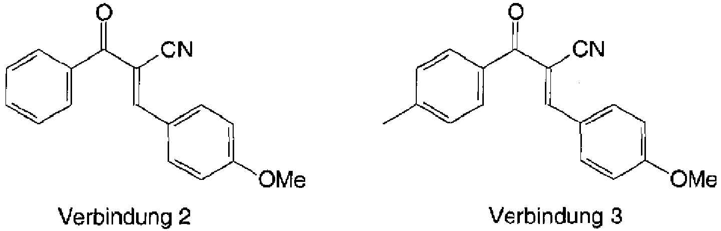 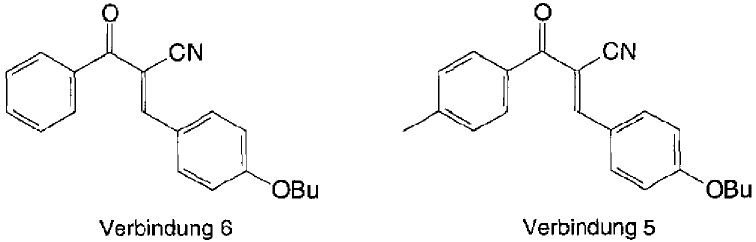 enthält.

8. Kosmetische Zubereitung nach einem der vorhergehenden Ansprüche, **dadurch gekennzeichnet, dass** die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat, Natriumcetearylsulfat, Kaliumcetylphosphat enthält.

## Claims

1. Cosmetic preparation which is in the form of an aqueous preparation or an O/W emulsion, comprising
a) particulate inorganic UV light protective filter pigments having an average particle size of 10 to 500 nm,
b) 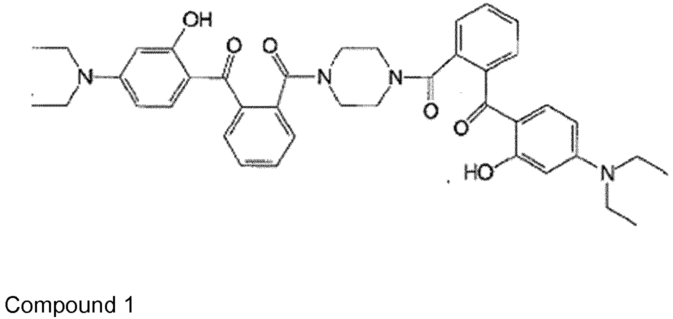 and
c) one or more compounds selected from the group of compounds comprising alpha-lipoic acid, folic acid, phytoene, D-biotin, coenzyme Q10, alpha-glucosylrutin, carnitine, carnosine, polidocanol, natural and/or synthetic isoflavonoids, flavonoids, creatine, creatinine, taurine, 3-alanine, tocopheryl acetate, urea; hyaluronic acid; dihydroxyacetone; 8-hexadecene-1,16-dicarboxylic acid and/or licochalcone A.

2. Preparation according to Claim 1, **characterized in that** the preparation comprises particulate inorganic light protective filter pigments at a concentration of 0.5 to 10% by weight, based on the total weight of the preparation.

3. Cosmetic preparation according to either of the preceding claims, **characterized in that** the preparation comprises compound 1 at a concentration of 0.25 to 10% by weight, based on the total weight of the preparation.

4. Cosmetic preparation according to any of the preceding claims, **characterized in that** the inorganic light protective filter pigments used are titanium dioxide, zinc oxide and/or iron oxide.

5. Preparation according to any of the preceding claims, **characterized in that** the inorganic light protective filter pigment used is titanium dioxide.

6. Preparation according to any of the preceding claims, **characterized in that** the particulate inorganic UV light protective filter pigments are surface-coated.

7. Preparation according to any of the preceding claims, **characterized in that** the preparation comprises further organic UV light protective filter pigments selected from the group of compounds comprising 2,2'-methylenebis(6-(2H-benzotriazol-2-yl)-4-(1,1,3,3-tetramethylbutyl)phenol); 2,4,6-tris(biphenyl)-1,3,5-triazine; 2,4,6-tris(terphenyl)-1,3,5-triazine;

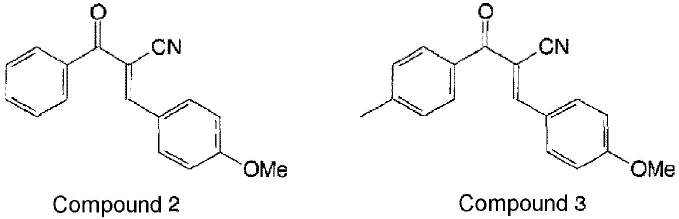

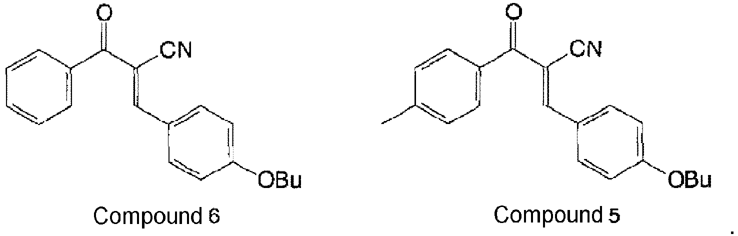

8. Cosmetic preparation according to any of the preceding claims, **characterized in that** the preparation comprises one or more O/W emulsifiers selected from the group of compounds comprising glyceryl stearate citrate, glyceryl stearate (self-emulsifying), stearic acid, stearate salts, polyglyceryl-3 methylglucose distearate, ceteareth-20, PEG-40 stearate, sodium cetearyl sulfate, potassium cetyl phosphate.

## Revendications

1. Préparation cosmétique qui se présente sous la forme d'une préparation aqueuse ou d'une émulsion H/E, contenant :
a) des pigments filtrants inorganiques particulaires de protection contre la lumière UV ayant une taille de particule moyenne de 10 à 500 nm,
b) 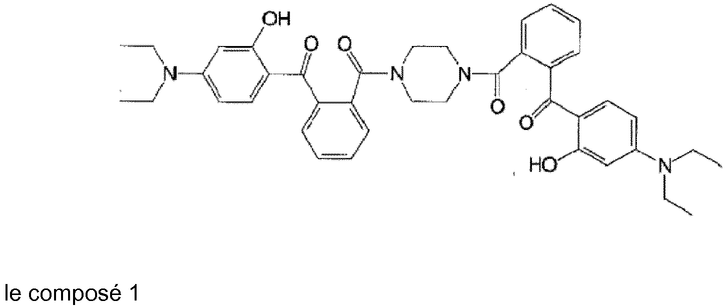 et
c) un ou plusieurs composés choisis dans le groupe constitué par les composés acide alpha-liponique, acide folique, phytoène, D-biotine, coenzyme Q10, alpha-glucosylrutine, carnitine, carnosine, polydocanol, isoflavonoïdes naturels et/ou synthétiques, flavonoïdes, créatine, créatinine, taurine, β-alanine, acétate de tocophéryle, urée, acide hyaluronique, dihydroxyacétone, acide 8-hexadécène-1,16-dicarboxylique et/ou licochalcone A.

2. Préparation selon la revendication 1, **caractérisée en ce que** la préparation contient des pigments filtrants inorganiques particulaires photoprotecteurs en une concentration de 0,5 à 10 % en poids, par rapport au poids total de la préparation.

3. Préparation cosmétique selon l'une quelconque des revendications précédentes, **caractérisée en ce que** la préparation contient le composé 1 en une concentration de 0,25 à 10 % en poids, par rapport au poids total de la préparation.

4. Préparation cosmétique selon l'une quelconque des revendications précédentes, **caractérisée en ce que** du dioxyde de titane, de l'oxyde de zinc et/ou de l'oxyde de fer sont utilisés en tant que pigments filtrants inorganiques photoprotecteurs.

5. Préparation cosmétique selon l'une quelconque des revendications précédentes, **caractérisée en ce que** du dioxyde de titane est utilisé en tant que pigment filtrant inorganique photoprotecteur.

6. Préparation cosmétique selon l'une quelconque des revendications précédentes, **caractérisée en ce que** les pigments filtrants inorganiques particulaires de protection contre la lumière UV sont revêtus en surface.

7. Préparation cosmétique selon l'une quelconque des revendications précédentes, **caractérisée en ce que** la préparation contient d'autres pigments filtrants organiques de protection contre la lumière UV choisis dans le groupe constitué par les composés 2,2'-méthylène-bis-(6-(2H-benzotriazol-2-yl)-4-(1,1,3,3-tétraméthylbutyl)-phénol) ; 2,4,6-tris-(biphényl)-1,3,5-triazine, 2,4,6-tris-(terphényl)-1,3,5-triazine ;

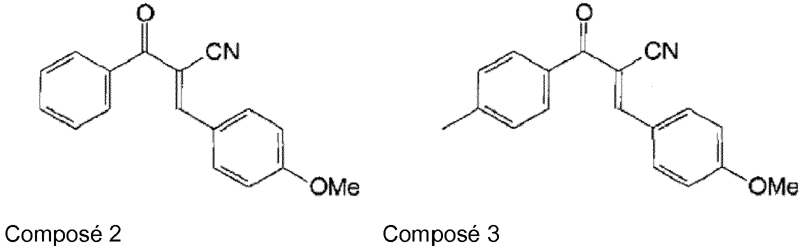

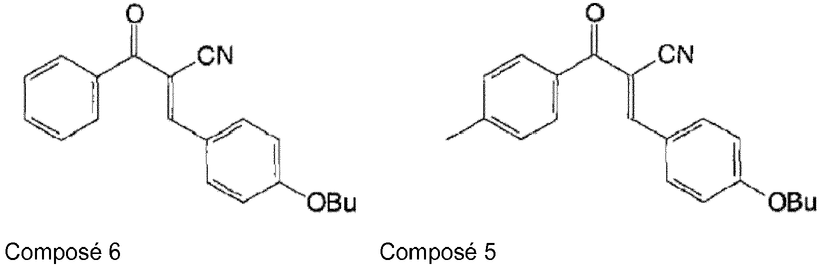

8. Préparation cosmétique selon l'une quelconque des revendications précédentes, **caractérisée en ce que** la préparation contient un ou plusieurs émulsifiant H/E choisis dans le groupe constitué par les composés stéarate-citrate de glycéryle, stéarate de glycéryle (auto-émulsifiant), acide stéarique, sels de stéarate, distéarate de polyglycéryl-3-méthylglycose, cétéareth-20, stéarate de PEG-40, sulfate de cétéaryle sodique, phosphate de cétyle potassique.
